# EUROPEAN PATENT APPLICATION

(11) **EP 3 663 297 A1**
(43) Date of publication of application: **10.06.2020**
(21) Application number: 18210798.7
(22) Date of filing: 06.12.2018
(51) Int. Cl.: C07D 493/08, C07D 307/88, C07D 307/89, C07C 61/09

(54) **DIELS-ALDER REACTION WITH FURANICS TO OBTAIN AROMATICS**

(71) Applicant: Nederlandse Organisatie voor toegepast- natuurwetenschappelijk onderzoek TNO, 2595 DA 's-Gravenhage (NL); Universiteit Utrecht Holding B.V., 3584 CM Utrecht (NL)
(72) Inventor: CROCKATT, Marc, 2595 DA 's-Gravenhage (NL); URBANUS, Johan, 2595 DA 's-Gravenhage (NL); BRUIJNINCX, Peter Cornelis Antonius, 3584 CM Utrecht (NL); LANCEFIELD, Christopher Stuart, 3584 CM Utrecht (NL); FÖLKER, Bart, 3584 CM Utrecht (NL)
(74) Representative: V.O.

(57) **Abstract**

The present invention is directed to the preparation of phthalic anhydride compounds and the intermediate phthalide compounds. In particular, the invention is directed to an improved bio-based route from furanic compounds to phthalic anhydride compounds by reacting furfuryl alcohol (*i.e*. 2-hydroxymethylfuran) or an analogue thereof having a nucleophilic atom on the 2-methyl, with a dienophile comprising an *α,β*-unsaturated carbonyl comprising an *α'*-leaving group. The inventions further involved preparation of phthalic anhydride compounds, phthalic acid compounds and reduction products of the intermediate phthalide compounds.

## Description

The invention is in the field of chemical processes. In particular, the invention is in the field of preparing aromatic compounds from bio-based furanic compounds.

In the recent years, interest in furanic compounds as intermediate chemical compounds for the production of chemicals from biomass has increased considerably. Of particular interest is the production of renewable aromatics from furanics by a cyclo-addition or Diels-Alder reaction with dienophiles. Depending on their specific substitution pattern, these aromatics can conveniently be used for production of a wide range of mass consumption products, such as plasticizers, synthetic fibers, (plastic bottles), fire-retardant materials, resins and the like. To this end, the reactions of a variety of different furanics with dienophiles have been investigated.

One target of interest is phthalide and its analogues and derivatives, such as phthalimides, phthalic diamides, phthalic anhydride and analogues, including phthalic acid and phthalate esters. One currently known bio-based route to phthalic anhydride involves the Diels-Alder reaction of furan (produced by decarbonylation of bio-based furfural, incurring CO loss) with maleic anhydride (Mahmoud, Green Chemisty, 16 (2014) 167-175). The produced Diels-Alder adduct can then be ring-opened and dehydrated using a mixture of methanesulfonic acid (expensive and difficult to recycle) and acetic anhydride, to produce phthalic anhydride. A drawback of this method is the poor atom economy, and a prohibitive processability and poor scalability of the final step resulting in a poor final yield. In addition, expensive, difficult to recycle regents are required.

The present inventors found an improved bio-based route from furanic compounds to phthalic anhydride compounds by reacting furfuryl alcohol (*i.e.* 2-hydroxymethylfuran) or an analogue thereof having a nucleophilic atom on the 2-methyl, with a dienophile comprising an *α,β-*unsaturated carbonyl comprising an *α*'-leaving group. Surprisingly, this reaction results in the clean formation of the phthalide as illustrated in Scheme 1, wherein the backbone of the furfuryl alcohol (*i.e.* 2-hydroxymethylfuran) or an analogue is illustrated by 'A', the nucleophilic atom by 'Nu', the dienophile by 'B', the *α*'-leaving group by 'LG' and the phthalide compound by 'D'.

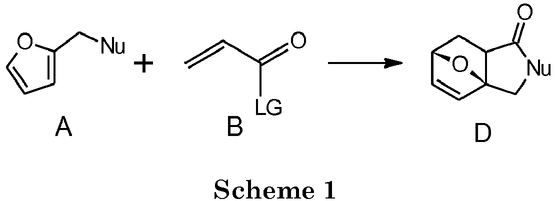

Thus, unlike the typically expected formation of a complex mixture of regio- and stereoisomers, a single compound can be predominantly formed. Without wishing to be bound by theory, the inventors believe that the reaction of 'A' and 'B' proceeds by formation of intermediates 'C1' and 'C2' and that a further reaction of 'C2' provides a sink such that the reaction equilibrium is pulled to a predominant, single product (as illustrated in Scheme 2).

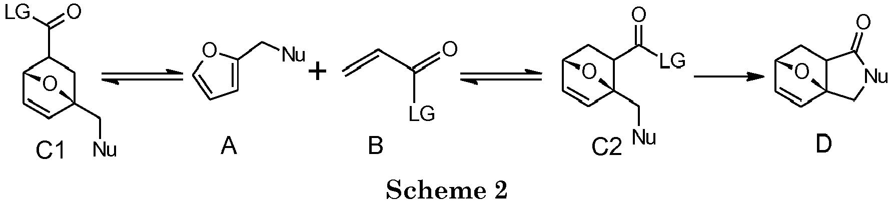

Accordingly, in a preferred embodiment, the present invention is directed to a method for preparing a phthalide compound precursor according to structure IV, comprising reacting a furanic compound according to structure I with a dienophile according to structure II, 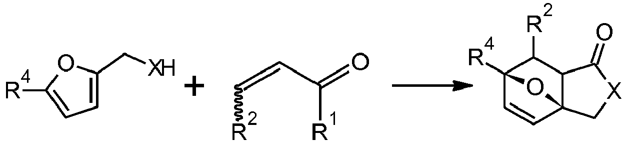 wherein
- X is selected from the group consisting of O, NH and S;
- R⁴ is selected from H, Me, CH₂OR⁵, CH₂NR⁵R⁶, CHO, CO₂H, CO₂R₅, CONR⁵R⁶, CR⁵=N-NR⁵R⁶, wherein
   R⁵ and R⁶ are independently selected from H, C₁-C₆ alkyl, C₆-C₁₂ aryl, C(O)R⁷, wherein R⁷ is selected from alkoxy, OH, NH₂ or a solid support, preferably H, Me, CH₂OH, CHO and CO₂H;
- R¹ is a leaving group, preferably selected from the group consisting of halide, O-EWG, NH-EWG and S-EWG, more preferably O-EWG, wherein EWG is an electron withdrawing group; and
- R² is selected from the group consisting of H, C₁-C₆ alkyl and C(Y)R³, wherein
- Y isone or two selected from the group consisting of H, halide, O (*e.g.* =O) and combinations thereof, preferably H,
- R³ is alkoxy, OH, NH₂, or C(Y)R³ is said O-EWG, NH-EWG or S-EWG when R¹ is O-EWG, NH-EWG or S-EWG such that R¹ and R² form, together with the three carbon atoms that connect these groups, a heterocycle, preferably a five-membered heterocycle.

In the present context, in case a formula used herein comprises a chiral center of which the chirality is not indicated, said formula is meant to illustrate all variations of the chirality of said chiral center and concomitantly is meant to illustrate all individual stereoisomeric compounds and mixtures thereof. In addition, in case a formula used herein comprises a chiral center of which the chirality is indicated, this indication of the chirality is meant to illustrate the relative chirality of the chiral center with respect to other stereogenic centers within the same formula or compound, unless explicitly indicated otherwise. Thus, unless explicitly described otherwise, the structural formulae that illustrate the compounds described herein, illustrate both enantiomers of said compounds and/or diastereoisomers thereof, if applicable. Accordingly, for sake of completeness it is noted that the word 'compound' in reference to a particular formula, may refer to a plurality of compounds, i.e. a mixture of several isomers such as enantiomers or diastereoisomers. In general, part of the precursor IV will have the relative stereochemistry according to the structure below (wherein X is indicated as the oxygen O). However, the treatment with acid of base could cause this to change.

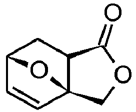

The wavy bond or squiggly bond symbol 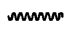 in Formula II indicates that the stereochemistry of the double bond may be *entgegen* (*E*), or *zusammen* (*Z*), or a mixture thereof.

The leaving group R¹ typically comprises an electron-withdrawing group as this is believed to facilitate the lactonization (*i.e.* the reaction of the XH group and the C(O)R¹ group). Typical activated esters, anhydrides (both mixed or symmetrical), activated amides, activated thio-esters, or acid halides as represented by the group consisting of halide, O-EWG, NH-EWG and S-EWG, wherein EWG is an electron withdrawing group are suitable. Activated esters include those typically used in peptide chemistry such as *N*-hydroxysuccinimide (NHS) esters and the like. The EWG can be known electron-withdrawing group such as a carbonyl, halide-substituted hydrocarbyls, nitrile-substituted hydrocarbyls, sulfonyl-substituted hydrocarbyls and nitro-substituted hydrocarbyls. A carbonyl as the EWG in O-EWG thus represents an anhydride as the dienophile. For instance, if EWG in O-EWG is acryloyl (*i.e.* C(O)CH=CH₂) and R² is H, the dienophile is the symmetrical acrylic anhydride. If EWG in O-EWG is formyl (*i.e.* C(O)CH=CH₂) and R² is H, the dienophile is the unsymmetrical (or mixed) acrylic formyl anhydride. A fluoride-substituted hydrocarbyl and/or a nitrophenol ester (*e.g.* 4-nitrophenol ester) is preferred as the EWG, as this is generally more electronegative than the alternatives. Examples of particularly preferred fluoride-substituted hydrocarbyls include 1,1,1,3,3,3-hexafluoroisopropyl or trifluoroethanol. The leaving group may optionally be linked to a solid support through suitable functionalization of the EWG.

For sake of clarity it is noted that R³ may be alkoxy, OH, NH₂, SH, or R³ is such that C(Y)R³ is said is O-EWG, NH-EWG or S-EWG when R¹ is O-EWG, NH-EWG or S-EWG such that R¹ and R² form, together with the three carbon atoms that connect these groups, a heterocycle, preferably a five-membered heterocycle. Thus, in this embodiment, R² is linked to the O, NH or S of R¹ by virtue of C(Y)R³ being said O-EWG, NH-EWG or S-EWG group. When C(Y)R³ is O-EWG, NH-EWG or S-EWG, C(Y) can be regarded as the EWG and R³ as the O, NH or S. In this respect it is also noted that in the embodiment wherein Y is one O, the group C(Y)R³ thus represents the carbonyl group C(=O)R³.

As explained herein above more generally, the present inventors found that this reaction typically proceeds through intermediate compounds IIIa and IIIb, and that XH likely intramolecularly reacts with the C(O)R¹ functionality in intermediate compound IIIa only.

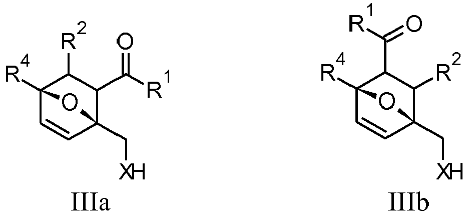

Generally, for sake of cost and energy efficiency, it is preferred to carry out the method of preparing the precursor according to structure IV in one single step and without isolation of one or more intermediate compounds such as compounds IIIa and/or IIIb. However, isolation of intermediate compound IIIa and/or intermediate compound IIIb, followed by further reacting said intermediate compound or compounds into the precursor according to structure IV is also regarded to be a particular embodiment of the present invention.

It was further found that the presence of a base when reacting the furanic and the dienophile is beneficial. Particularly, weak bases are preferred and weak bases having a pK_{b} in water of between 2 and 12 are more preferred. Even more preferable the base has a pK_{b} between 4 and 10, most preferably between 6 and 9. Examples of suitable bases included non-nucleophilic bases (which are preferred) such as bicarbonates, acetates, dichloroacetate and triethylamine with which all good yields have been obtained.

Another reaction condition of the method generally pertains contacting the furanic compound and the dienophile in a ratio of between 5:1 to 1:5, more preferably in a ratio between 2:1 to 1:2, most preferably in a ratio of about 1.1:1 to 1:1.1 such as about 1:1. The reaction of the furanic compound and the dienophile is typically carried out at a temperature below 200 °C, more preferably below 130°C, most preferably in the range of 0 to 100 °C.

To aid in the activation of the reaction, it may be preferred to carry out the method in a solvent. However, in alternative embodiments, reacting the furanic compound and the dienophile is carried out essentially neat or entirely neat.

The method of the present invention is preferably carried out in a continuous fashion. Reaction conditions, set-ups and reactors that may particularly be suitable in this respect may be those described in European patent application no. 18170098.0, which is herein incorporated in its entirety.

The phthalide compound precursor of the present invention is particular suitable to prepare a phthalide compound in accordance with formula V. This preparation comprises ring-opening and aromatization of the precursor according to structure IV.

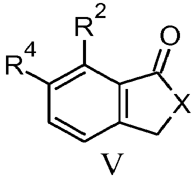

The ring-opening and aromatization can be carried out by contacting the precursor with an acid, including Bronsted and Lewis acids and soluble and solid acids, preferably an acid selected from the group consisting of methanesulfonic acid, sulfuric acid, acidic ion exchange resins, zeolites, optionally in combination with an activating agent. Typically, many acids will be suitable for this reaction due to the relative stability of the intermediate IV. Generally suitable conditions are those disclosed in European patent application no. 18170098.0 as well. The activating agent is selected from the group consisting of acylating agent, triflating agent, sulfonating agent, carbamylating agent, carbonylating agent, or combinations thereof, preferably the activating agent is an acylating agent, more preferably an acylating agent selected from the group consisting of acetic anhydride, acetyl chloride, propionic anhydride, butyric anhydride, isobutyric anhydride, trimethylacetic anhydride, mixed anhydrides thereof, or combinations thereof, most preferably the activating agent comprises acetic anhydride.

Alternatively to the use of an acid, the precursor can be ring-opened with base (*i.e.* by deprotonation of the proton alpha to the lactone carbonyl in IV).

In a particular embodiment of the present invention, the phthalide compound is oxidized to phthalic anhydride compound according to formula VI using conventional methods, such as those processes including nitric acid, those conditions known in the Amoco process, enzymatic oxidation, electrochemical oxidation, and the like. The phthalic anhydride compound according to formula VI can subsequently be hydrolyzed to phthalic acid compound according to formula VII, or esters, amides and imides thereof using conventional methods. Alternatively, these processes can be carried out vice-versa: phthalide compound of formula V can first be hydrolyzed, which can be followed by oxidation and derivatization to result in the phthalic acid compound and its derivatives.

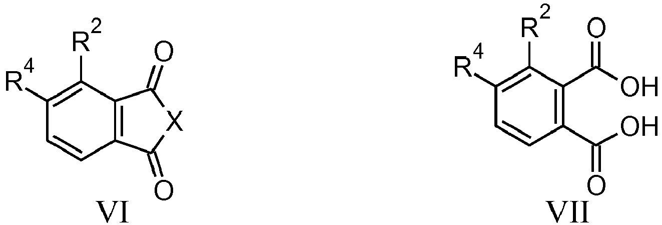

In a particular embodiment of the present invention, the phthalide compound precursor is reduced instead of oxidized as described above. Reduction can for instance result in any of compounds IIX and IX.

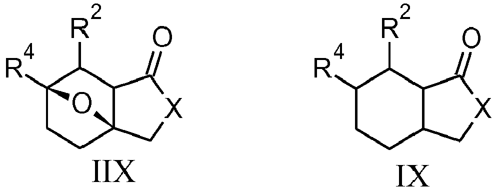

Typical reduction conditions include those for hydrogenation using a known catalyst such as Pd/C. According to the present invention, the phthalide compound precursor may also be used to prepare any of the compounds X, XI, XII and XIII.

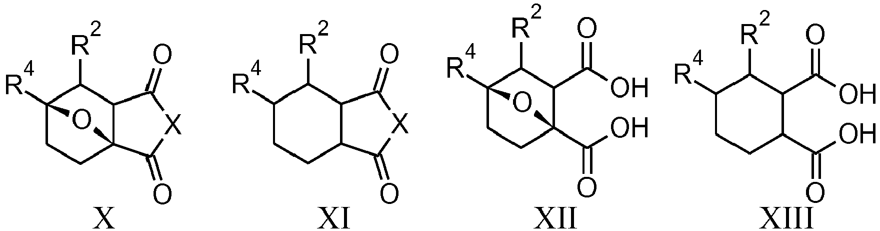

In general, the present method allows the preparation of the compounds V, VI, IIX, IX, X and XI from compound IV according to the following Scheme 3.

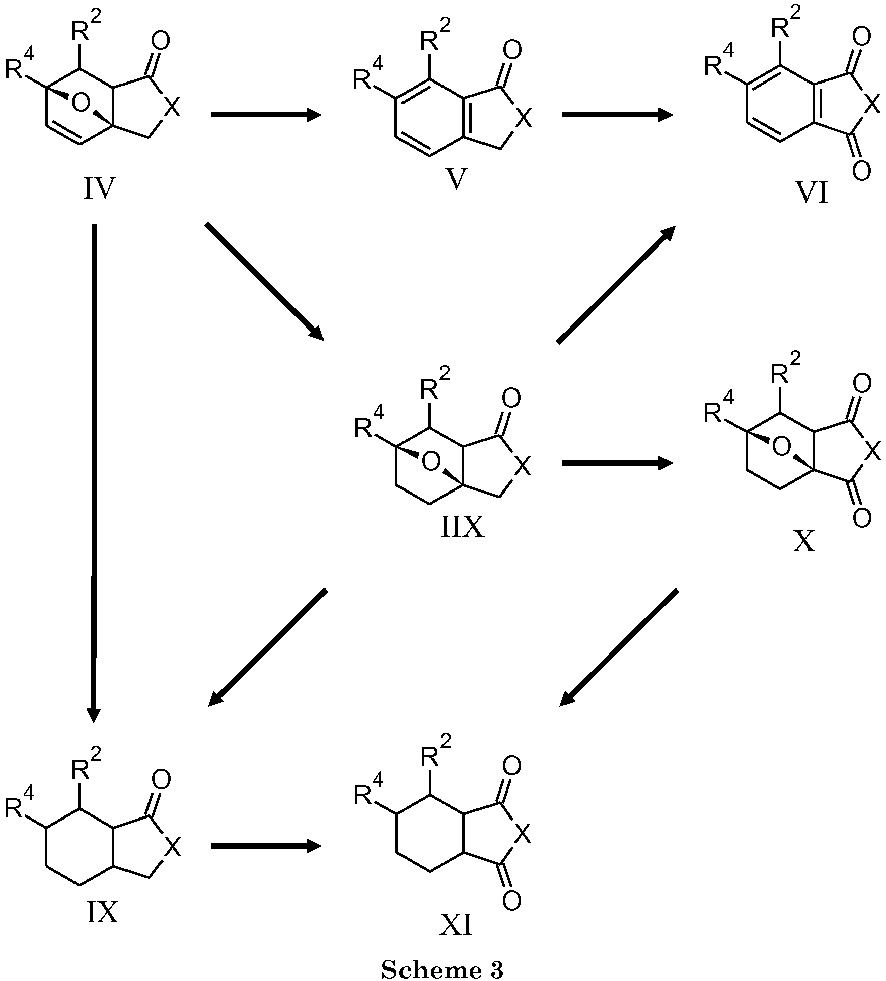

For example, the above-mentioned compounds X, XI, XII and XIII can be obtained by a method comprising oxidation of the compound in accordance with formula IIX (resulting in the compound of formula X), oxidation of the compound in accordance with formula IX (resulting in the compound of formula XI), oxidation and then reduction of the compound in accordance with formula IIX (resulting in the compound of formula XI, see also WO/2016099275) ;oxidation of the compound in accordance with formula IIX followed by hydrolysis (resulting in the compound of formula XII) or oxidation of the compound in accordance with formula IIX followed by hydrolysis (resulting in the compound of formula XIII).

For sake of clarity and conciseness, in the context of the present invention, the phthalide compound precursors are collectively referred to with the term phthalide, even if X may also include NH or S (besides O) in formula IV. Similarly, the phthalide compound according to formula V that may be obtained from said precursor includes compound wherein X is NH or S (besides O). Thus, the term phthalide compound includes phthalimides and thiophthalide and the like. The same applies to, *mutatis mutandis,* the phthalic anhydride compounds disclosed herein.

As used herein, the singular forms "a", "an" and "the" are intended to include the plural forms as well, unless the context clearly indicates otherwise. The term "and/or" includes any and all combinations of one or more of the associated listed items. It will be understood that the terms "comprises" and/or "comprising" specify the presence of stated features but do not preclude the presence or addition of one or more other features.

For the purpose of clarity and a concise description features are described herein as part of the same or separate embodiments, however, it will be appreciated that the scope of the invention may include embodiments having combinations of all or some of the features described.

The present invention can be illustrated by the following examples.

### Example 1 - 1,1,1,3,3,3-Hexafluoroisopropylacrylate

A suspension of 1,1,1,3,3,3-hexafluoroisopropanol (49.91 g, 297 mmol, 1.2 eq.) and scandium(III) triflate (0.10 g, 0.20 mmol, 0.08 mol %) were stirred at 20 °C and acryloyl chloride (22.40 g, 248 mmol, 1 eq.) was added dropwise over the course of 5 minutes. After stirring the mixture for 20 hours at 20 °C, sodium bicarbonate was added to the stirred suspension until pH > 7 was achieved. The solids were removed by filtration, and filtrates were distilled under nitrogen to yield the desired product.

### Example 2- 7,7a-Dihydro-3H-3a,6-epoxyisobenzofuran-1(6H)-one

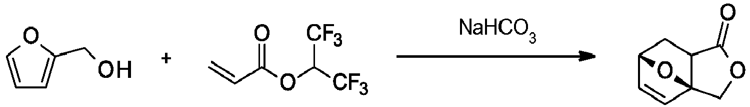

A suspension of furfuryl alcohol (leq.), 1,1,1,3,3,3-hexafluoroisopropylacrylate (1.1eq.) and sodium bicarbonate (1 mol%) was stirred at 80 °C for 22 hours then the mixture was cooled to 20°C and the product was isolated as a solid (64% yield) following flash chromatography.

### Example 3 - 7,7a-Dihydro-3H-3a,6-epoxyisobenzofuran-1(6H)-one

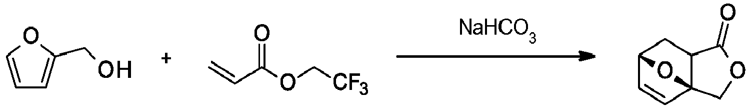

A suspension of furfuryl alcohol (leq.), 1,1,1-trifluoroethylacrylate (leq.) and sodium bicarbonate (20 mol%) were stirred at 80 °C for 22 hours. This yielded 7,7a-Dihydro-3H-3a,6-epoxyisobenzofuran-1(6H)-one (33% yield, 39% selectivity).

### Example 4 - 7,7a-Dihydro-3H-3a,6-epoxyisobenzofuran-1(6H)-one

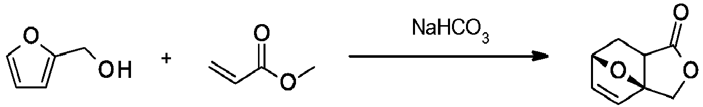

A suspension of furfuryl alcohol (leq.), methyl acrylate (leq.) and sodium bicarbonate (20 mol%) were stirred at 80 °C for 22 hours. This yielded 7,7a-Dihydro-3H-3a,6-epoxyisobenzofuran-1(6H)-one (2% yield, 8% selectivity).

### Example 5 - 7,7a-Dihydro-3H-3a,6-epoxyisobenzofuran-1(6H)-one

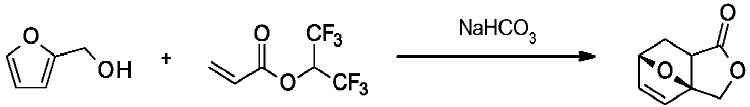

A suspension of furfuryl alcohol (leq.), 1,1,1,3,3,3-hexafluoroisopropylacrylate (1.5eq.) and sodium bicarbonate (20 mol%) was stirred at 80 °C for 22 hours. This yielded 7,7a-Dihydro-3H-3a,6-epoxyisobenzofuran-1(6H)-one (68% yield, 88% selectivity).

### Example 6 - 7,7a-Dihydro-3H-3a,6-epoxyisobenzofuran-1(6H)-one

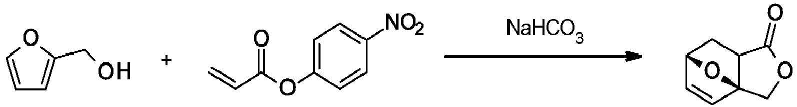

A suspension of furfuryl alcohol (leq.), 4-nitrophenol acrylate (1.1eq.) and sodium bicarbonate (2 mol%) was stirred at 80 °C for 36 hours. This yielded 7,7a-Dihydro-3H-3a,6-epoxyisobenzofuran-1(6H)-one (86% yield, 91% selectivity).

### Example 7 - 7,7a-Dihydro-3H-3a,6-epoxyisobenzofuran-1(6H)-one

A suspension of furfuryl alcohol (leq.) and acrylic anhydride (leq.) were stirred at 45°C for 16 hours. This yielded 7,7a-Dihydro-3H-3a,6-epoxyisobenzofuran-1(6H)-one (43% yield, 44% selectivity).

### Example 8 - Screening of Conditions for Diels-Alder Lactonisation to 7,7a-Dihydro-3H-3a,6-epoxyisobenzofuran-1(6H)-one

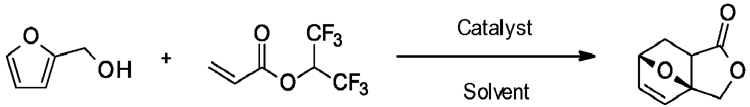

A mixture of furfuryl alcohol (leq.), 1,1,1,3,3,3-hexafluoroisopropylacrylate (leq.), catalyst (see Table X) and solvent (see Table 1; concentration = 1.1 M) was stirred at the stated temperature (see Table 1). The results of these reactions are shown in Table 1.

**Table 1**

| **Solvent** | **Cat.** | **mol% Cat.** | **T (°C)** | **Yield (%)** | **Selectivity (%)** |
|---|---|---|---|---|---|
| Neat | NaHCO₃ | 2 | 60 | 34 | 43 |
| | | 20 | 60 | 17 | 20 |
| | | 0.5 | 80 | 38 | 70 |
| | | 1 | 80 | 64 | 82 |
| | | 2 | 80 | 54 | 66 |
| | | 20 | 80 | 26 | 28 |
| | NaCH₃CO₂ | 2 | 80 | 44 | 55 |
| | | 20 | 80 | 23 | 27 |
| | NaCHCl₂CO₂ | 2 | 80 | 31 | 50 |
| | | 20 | 80 | 45 | 58 |
| | NEt₃ | 2 | 80 | 30 | 34 |
| EtOAc | NaHCO₃ | 2 | 80 | 43 | 83 |

### Example 9 - Screening of Acid Conditions for Aromatization to Phthalide

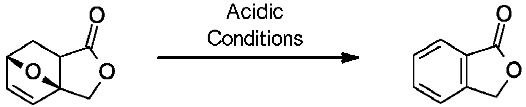

The lactone (leq.) is added to a stirred solution of the desired acid (See Table Y), in solvent (where applicable - See Table 2 - all reactions with solvent performed at 0.33 M) at 20 °C, then the mixture is heated to the stated temperature (see Table 2) for 1 hour. The results of these reactions are shown in Table Y.

**Table 2**

| **Solvent** | **Cat.** | **T (°C)** | **Yield (%)** |
|---|---|---|---|
| Neat | MSA* (13eq.) | 20 | 66 |
| Toluene | MSA (0.1eq.) | 80 | 66 |
| | Hf(OTf)₄ (0.1eq.) | 80 | 60 |
| | TfOH (0.1eq.) | 80 | 63 |
| | TfOH (0.01eq.) | 80 | 63 |
| | Silica-TfOH (0.01eq.) | 80 | 58 |
| CHCl₃ | TfOH (0.1eq.) | 80 | 79 |
| AcOH | TfOH (0.1eq.) | 80 | 56 |

| | | | |
|---|---|---|---|
| * MSA: methanesulfonic acid | | | |

### Example 10 - Screening of Dry, Assisted Acid Conditions for Aromatization to Phthalide

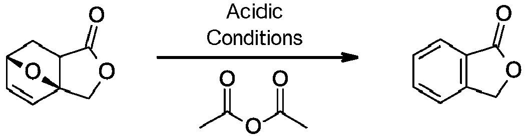

The lactone (leq.) is added to a stirred solution of the desired acid (See Table Z) and acetic anhydride (See Table Z), in solvent (where applicable - See Table Z - all reactions with solvent performed at 1 M), at 20 °C, then the mixture is heated to the stated temperature (see Table 3). The results of these reactions are shown in Table 3.

**Table 3**

| **Solvent** | **Cat.** | **AC₂O** | **T (°C)** | **Yield (%)** |
|---|---|---|---|---|
| Neat | MSA (13eq) | 20 vol% | 20 | 75 |
| | MSA (0.5 eq.) | 4 eq. | 20 | 97 |
| | MSA (0.5 eq.) | 4 eq. | 80 | 98 |
| | MSA (0.1 eq.) | 4 eq. | 80 | 95 |
| | MSA (0.02 eq.) | 4 eq. | 80 | 94 |
| | MSA (0.01 eq.) | 4 eq. | 80 | 39 |
| | TfOH (0.01 eq.) | 4 eq. | 80 | 95 |
| | H₂SO₄ (0.01 eq.) | 4 eq. | 80 | 80 |
| | Amberlyst-15 (0.1 eq) | 4 eq. | 80 | 82 |
| EtOAc | Amberlyst-15 (0.1 eq) | 4 eq. | 80 | 78 |
| | Amberlyst-15 (0.1 eq) | 2 eq. | 80 | 89 |

## Claims

1. Method for preparing a phthalide compound precursor according to structure IV, comprising reacting a furanic compound according to structure I with a dienophile according to structure II, 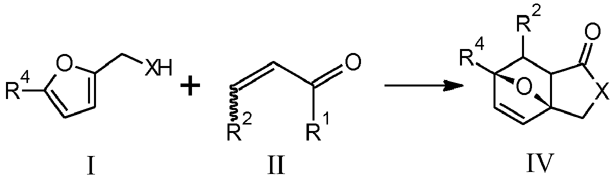 wherein
- X is selected from the group consisting of O, NH and S;
- R⁴ is selected from H, Me, CH₂OR⁵, CH₂NR⁵R⁶, CHO, CO₂R₅, CONR⁵R⁶, CR⁵=N-NR⁵R⁶, wherein
R⁵ and R⁶ are independently selected from H, C₁-C₆ alkyl, C₆-C₁₂ aryl, C(O)R⁷, wherein R⁷ is selected from alkoxy, OH, NH₂ or a solid support;
- R¹ is a leaving group, preferably selected from the group consisting of halide, O-EWG, NH-EWG and S-EWG, more preferably O-EWG, wherein EWG is an electron withdrawing group; and
- R² is selected from the group consisting of H, C₁-C₆ alkyl and C(Y)R³, wherein
- Y is one or two selected from the group consisting of H, halide, O and combinations thereof, preferably H,
- R³ is alkoxy, OH, NH₂, SH, or, when R¹ is O-EWG, NH-EWG or S-EWG, said R³ is such that C(Y)R³ is said O-EWG, NH-EWG or S-EWG such that R¹ and R² form a heterocycle.

2. Method according to the previous claim, wherein X is O, R⁴ is H, and/or R² is H.

3. Method according to any of the previous claims, wherein EWG is selected from the group consisting of carbonyl, halide-substituted hydrocarbyls, nitrile-substituted hydrocarbyls, sulfonyl-substituted hydrocarbyls and nitro-substituted hydrocarbyls, preferably a fluoride-substituted hydrocarbyl, more preferably 1,1,1,3,3,3-hexafluoroisopropyl or trifluoroethanol and optionally linked to a solid support.

4. Method according to any of the previous claims, wherein the furanic and the dienophile are reacted in the presence of a base, preferably a weak base, more preferably a weak base having a pK_{b} in water of between 2 and 12, even more preferably between 4 and 10, most preferably between 6 and 9.

5. Method according to any of the previous claims, said method comprising contacting the furanic compound and the dienophile in a ratio of between 5:1 to 1:5, more preferably in a ratio between 2:1 to 1:2, most preferably in a ratio of about 1.1:1 to 1:1.1 such as about 1:1.

6. Method according to any of the previous claims, wherein reacting the furanic compound and the dienophile is carried out at a temperature below 200 °C, more preferably below 130°C, most preferably in the range of 0 to 100 °C.

7. Method according to any of the previous claims, wherein reacting a furanic according to structure I with a dienophile according to structure II proceeds through intermediate compound IIIa and/or intermediate compound IIIb.

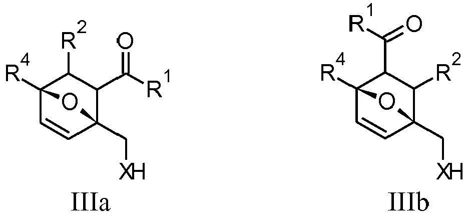

8. Method according to any of the previous claims, wherein preparing the precursor according to structure IV is carried out in one single step and without isolation of one or more intermediate compounds.

9. Method according to claim 6, comprising isolation of intermediate compound IIIa and/or intermediate compound IIIb, followed by further reacting said intermediate compound or compounds into the precursor according to structure IV.

10. Method for the preparation of any of the compounds in accordance with formulae V, VI, VII, IIX, IX, X, XI, XII and XIII, or an ester, amide or imide thereof, 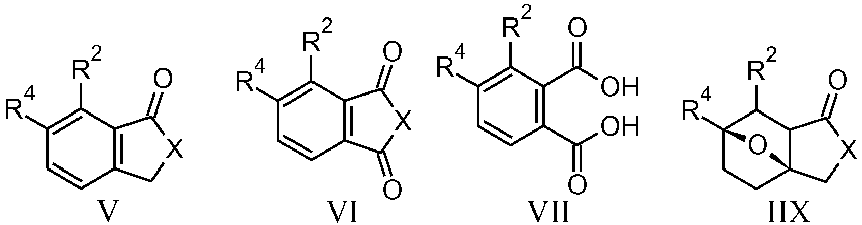 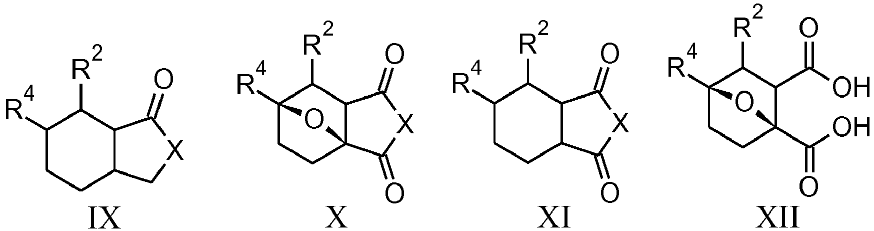 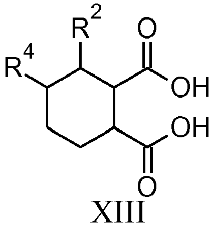 comprising reacting the precursor according to structure IV in one or more further reactions, wherein the precursor according to structure IV is preferably prepared with the method according to any of the previous claims.

11. Method for the preparation of a phthalide compound in accordance with formula V, comprising ring-opening and aromatization of the precursor according to structure IV, 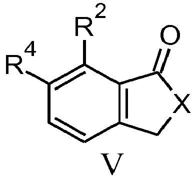 which precursor is preferably prepared in a method according to any of claims 1-9.

12. Method according to claim 11, wherein the ring-opening and aromatization is carried out by contacting the precursor with an acid, preferably an acid selected from the group consisting of methanesulfonic acid, sulfuric acid, acidic ion exchange resins, zeolites, optionally in combination with an activating agent such as acetic acid anhydride.

13. Method for the preparation of a phthalic anhydride compound according to formula VI, said method comprising preparation of the phthalide compound corresponding to formula V in accordance with any of claims 11-12, followed by oxidation of said phthalide compound to the phthalic anhydride compound.

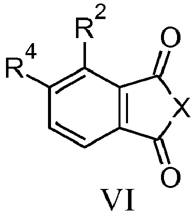

14. Method for the preparation of a phthalic acid compound according to formula VII or an ester, amide or imide thereof, comprising preparation of the phthalic anhydride compound corresponding to formula VI in accordance with claim 13, followed by hydrolysis of said phthalic anhydride compound or by hydrolysis of the phthalide compound in accordance with formula V in accordance with claim 11, followed by oxidation.

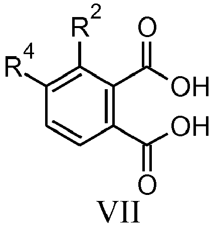

15. Method for the preparation of a compound in accordance with formulae IIX and/or IX, comprising reduction of the phthalide analogue precursor according to structure IV, 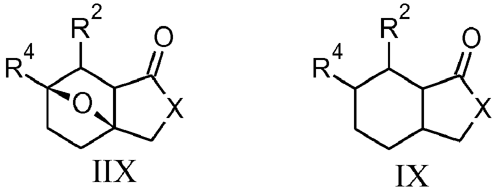 which phthalide analogue precursor is preferably prepared in a method according to any of claims 1-9.

16. Method for the preparation of a compound in accordance with:
- formula X, comprising oxidation of the compound in accordance with formula IIX, preferably prepared according to claim 15;
- formula XI comprising oxidation of the compound in accordance with formula IX, preferably prepared according to claim 15;
- formula XI, comprising oxidation and then reduction of the compound in accordance with formula IIX, preferably prepared according to claim 15;
- formula XII, comprising oxidation of the compound in accordance with formula IIX followed by hydrolysis, preferably prepared according to claim 15; or
- formula XIII, comprising oxidation of the compound in accordance with formula IX followed by hydrolysis, preferably prepared according to claim 15.

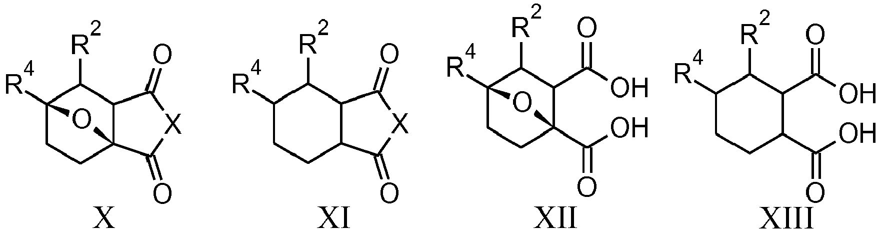

17. Compound according to any of formulae IIIa, IIIb, IIX, IX, X, XI, XII and XIII or an ester, amide or imide thereof.
